# FASCICULE DE BREVET EUROPEEN

(11) **EP 0 620 254 B1**
(45) Date de publication et mention de la délivrance du brevet: **04.09.1996**
(21) Numéro de dépôt: 94400764.0
(22) Date de dépôt: 08.04.1994
(51) Int. Cl.: C09C 1/00, C09C 3/06, C08K 3/00, C04B 33/14, C09D 11/00, B32B 9/00, C09D 7/12, D21H 17/67, A61K 7/00

(54) **Compositions à base de sulfures de terres rares, procédé de synthèse et utilisations**
Zusammensetzungen auf Basis von Sulfiden von seltenen Erden, Syntheseverfahren und Anwendungen
Compositions based on sulfides of rare earths, process for their synthesis and their use

(30) Priorité: 16.04.1993 FR 9304544
(43) Date de publication de la demande: 19.10.1994
(73) Titulaire: RHONE-POULENC CHIMIE, 92408 Courbevoie Cédex (FR)
(72) Inventeur: Chopin, Thierry, F.93200 Saint-Denis (FR); Dupuis, Dominique, F-95170 Deuil-La Barre (FR)
(74) Mandataire: Dubruc, Philippe

(56) Documents cités:
- EP-A- 0 203 838
- US-A- 4 545 967

## Description

La présente invention concerne, à titre de produits industriels nouveaux et utiles, des compositions inorganiques colorés à base de sulfures de terres rares. Plus précisément encore, elle concerne de nouvelles compositions à base de sesquisulfures de terres rares particuliers et d'oxydes transparents, lesdites compositions se présentant sous la forme de particules de type coeur-écorce (ou core-shell), les sulfures en constituant le coeur et les oxydes transparents l'écorce.

L'invention concerne également un procédé pour l'obtention de telles compositions, ainsi que certaines des utilisations possibles de ces dernières pour la coloration de divers matériaux.

Les pigments minéraux de coloration sont déja largement utilisés dans de nombreuses industries notamment dans celles des peintures, des matières plastiques et des céramiques. Dans de telles applications, les propriétés que sont, entre autres, la couleur intrinsèque, le pouvoir de coloration et le pouvoir opacifiant, la stabilité thermique, la stabilité chimique, la dispersabilité (aptitude d'un produit à se disperser correctemment dans un milieu donné), et l'absence de toxicité, constituent autant de critères particulièrement importants à prendre en considération dans le choix d'un pigment convenable.

Les sesquisulfures de terres rares, de formule générale M₂S₃ dans laquelle M désigne au moins une terre rare, sont des produits déja connu en soi et ont fait l'objet de nombreuses publications.

Par ailleurs, leur utilisation à titre de pigments colorés pour la coloration de différents matériaux, comme par exemple les plastiques, les peintures et autres, a déja été décrite notamment dans la demande de brevet EP-A- 0 203 838, au nom de la Demanderesse, et dont l'enseignement est ici inclus à titre de référence.

Toutefois, pour cette application, il s'avère que les sesquisulfures de terres rares connus à ce jour présentent des caractéristiques, notamment chromatiques, ainsi qu'une stabilité tant thermique que chimique, encore insuffisantes pour connaitre un réel développement.

Une autre difficulté réside également dans le fait que la plupart des procédés de synthèse visant à l'obtention de sesquisulfures de terres rares à haute pureté phasique mettent en oeuvre des réactions du type solide (composés de terres rares, tels qu'oxydes) - gaz (agents sulfurants, tels que H2S) dans des conditions de pressions et/ou de températures qui sont peu, voire pas du tout, compatibles avec une exploitation à l'échelle industrielle, et l'on évite en outre rarement la formation de certaines phases parasites constituées notamment d'oxysulfures de terres rares qui peuvent masquer ou dégrader la couleur intrinsèque des sesquisulfures purs désirés.

La présente invention a notamment pour but de résoudre les problèmes ci-dessus.

Plus précisémment encore, la présente invention vise à proposer une nouvelle famille de compositions inorganiques présentant, entre autres avantages, des caractéristiques chromatiques tout à fait remarquables, une stabilité chimique et thermique améliorées, ainsi qu'une absence de toxicité, lesdites compositions convenant en outre pour la coloration de nombreux matériaux.

Elle vise également à proposer un procédé permettant d'accéder de manière simple, économique, reproductible, contrôlée et compatible avec une production à l'échelle industrielle, à cette nouvelle famille de compositions.

A ces fins, il a maintenant été trouvé, et c'est ce qui constitue l'un des objets de la présente invention, de nouvelles compositions inorganiques colorés, lesdites compositions étant caractérisées par le fait qu'elles comprennent :
- un support (ou coeur, ou noyau) à base d'au moins un sesquisulfure de terres rares de formule M₂S₃ dans laquelle M représente au moins un élément pris dans le groupe constitué par les lanthanides de numéro atomique compris inclusivement entre 57 et 71 et l'yttrium, ledit support contenant en outre au moins un élément alcalin et/ou alcalino-terreux dont une partie au moins est incluse dans le réseau cristallin dudit sesquisulfure de terres rares,
- et une couche (ou écorce, ou enveloppe) à base d'au moins un oxyde transparent déposée à la surface dudit support et enrobant ce dernier.

La Demanderesse a trouvé que les nouvelles compositions ci-dessus pouvaient être utilisées avec succès en tant que pigments colorés en raison de leurs nombreuses et multiples propriétés avantageuses, telles que notamment un très large éventail d'excellentes couleurs intrinsèques, un très bon pouvoir de coloration, un très bon pouvoir opacifiant, une très bonne dispersabilité notamment dans les plastiques, une très bonne stabilité thermique à des températures pouvant aller jusqu'à 500°C, ainsi qu'une bonne stabilité chimique dans des milieux comme l'eau (à pH neutre, basique ou faiblement acide) et les solvants organiques.

On notera d'ores et déja que les supports constitutifs des compositions selon l'invention peuvent contenir un ou plusieurs alcalins, un ou plusieurs éléments alcalino-terreux, ou bien encore des mélanges entre alcalin(s) et alcalino-terreux.

Aussi, dans ce qui suit, l'expression "élément dopant" a été utilisée par simplicité pour désigner tant un métal alcalin qu'un métal alcalino-terreux, et elle doit s'entendre comme couvrant toute combinaison entre alcalin(s) et/ou alcalino-terreux, comme souligné ci-dessus.

Par ailleurs, chaque fois qu'utilisée dans l'exposé qui suit de la présente invention, la dénomination terre(s) rare(s) doit s'entendre comme désignant et couvrant, isolément ou en mélange, l'ensemble des éléments appartenant à la famille des lanthanides ayant un numéro atomique compris inclusivement entre 57 et 71, ainsi que l'yttrium de numéro atomique 39.

Le procédé de synthèse des compositions selon l'invention constitue un second objet de la présente invention, et il se caractérise par le fait qu'il comprend les étapes essentielles suivantes:
(i) on met en contact un support minéral tel que ci-dessus défini et un précurseur de l'oxyde transparent désiré,
(ii) on précipite l'oxyde transparent,
(iii) et enfin on sépare la composition obtenue du milieu de réaction.

Globalement, le procédé selon l'invention repose sur le principe d'une précipitation chimique d'un précurseur d'oxyde transparent opérée "in situ" dans un milieu constitué d'une dispersion (ou suspension) du support minéral précité, ledit oxyde précipité se déposant alors à la surface de chacune des particules dudit support minéral.

Il en résulte l'obtention directe dans le milieu de réaction de particules composites (ou pigments) qui correspondent au support originel mais sous une forme parfaitement encapsulée par une couche protectrice externe, d'épaisseur contrôlée, d'oxyde transparent.

Le procédé selon l'invention présente pour avantage, entre autres, de ne pas ou peu perturber d'une part la granulométrie des particules constituant le support initial avant enrobage, ainsi que, d'autre part, les excellentes caractéristiques chromatiques intrinsèques attachées à ces dernières, lesdites caractéristiques pouvant même s'en trouver sensiblement améliorées; en outre, I'écorce ainsi constituée à la surface desdites particules initiales permet d'aboutir à des compositions dont les propriété de stabilité thermique et chimique sont nettement améliorées.

Enfin, un dernier objet de l'invention concerne des pigments colorés qui sont caractérisés par le fait qu'ils sont constitués de ou qu'ils sont à base de compositions du type mentionné plus haut.

Mais d'autres caractéristiques, aspects et avantages de l'invention apparaitront encore plus clairement à la lecture de la description détaillée qui va suivre, ainsi que des divers exemples destinés à l'illustrer de manière concrète mais nullement limitative.

On commencera par décrire les nouvelles compositions selon l'invention. Une première caractéristique essentielle des compositions à base de sesquisulfure de terre rare selon l'invention réside dans leur structure particulière.

Comme déja souligné ci-avant, cette structure peut être décrite de la manière suivante : la composition selon l'invention comporte un support ou particule minérale qui forme un noyau et, enrobant ce noyau, une couche périphérique d'oxyde transparent.

Bien entendu, certaines variantes autour de cette structure sont possibles. En particulier, la couche périphérique enrobant la particule peut ne pas être parfaitement continue ou homogène. Toutefois, de préférence, les compositions selon l'invention sont constitués d'une couche de revêtement uniforme et d'épaisseur contrôlée d'oxyde transparent, et ceci de manière à ne pas altérer la couleur originelle du support avant enrobage.

Une deuxième caractéristique essentielle des compositions à base de sesquisulfure de terre rare selon l'invention réside dans leur composition particulière, et ceci tant au niveau du support minéral lui-même que de la couche protectrice qui l'enrobe.

Ainsi, le premier élément spécifique constitutif des compositions à base de sesquisulfure de terre rare selon l'invention est tout d'abord l'oxyde transparent déposé à la surface du support. Par oxyde transparent, on entend ici un oxyde qui, une fois déposé sur un support sous la forme d'une pellicule plus ou moins fine, n'absorbe que peu ou pas du tout les rayons lumineux dans le domaine du visible, et ceci de manière à ne pas ou peu masquer la couleur intrinsèque d'origine dudit support. En outre, il convient de noter que le terme oxyde, qui est utilisé par commodité dans l'ensemble de la présente descripition, doit être entendu comme couvrant également des oxydes de type hydratés.

Ces oxydes, ou oxydes hydratés, peuvent être amorphes et/ou cristallisés.

A titre d'exemple de tels oxydes, on peut plus particulièrement citer l'oxyde de silicium (silice), l'oxyde d'aluminium (alumine), l'oxyde de zirconium (zircone), l'oxyde de titane, le silicate de zirconium ZrSiO₄ (zircon) et les oxydes de terres rares. Parmi ceux-ci, on préfére mettre en oeuvre ceux ne présentant pas de toxicité officiellement reconnue pour l'homme et/ou son environnement.

Selon un mode préféré de réalisation des compositions selon la présente invention, la couche enrobante est à base de silice. De manière encore plus avantageuse, cette couche est essentiellement, et de préférence uniquement, constituée de silice.

Le deuxième élément spécifique constitutif des compositions à base de sesquisulfure de terre rare selon l'invention est ensuite le support minéral à base de sesquisulfures de terres rares dopés par un ou plusieurs éléments alcalins et/ou alcalino-terreux. Comme cela ressortira clairement de l'exposé ci-dessous, un tel support, avant son enrobage, constitue déja en lui-même un pigment minéral coloré auquel sont attachées de nombreuses propriétés avantageuses, et un procédé de synthèse particulier permettant d'accéder à ce type de compositions originales sera quant à lui développé en détails par la suite. Ledit support minéral coloré détermine et conditionne pour l'essentiel la coloration finale de la composition ou du pigment composite coloré de la présente invention; en d'autres termes, l'obtention d'une composition ou d'un pigment composite conforme à l'invention présentant une couleur donnée passe par l'utilisation d'un support présentant cette même couleur. La couche périphérique d'oxyde transparent permet de ne pas, ou peu, altérer la couleur originel du support, tout en améliorant la résistance thermique et/ou chimique de ce dernier.

L'élément dopant peut être présent sous différentes formes dans le support.

Toutefois, selon l'invention, il est de préférence essentiellement présent sous une forme combinée avec les sesquisulfures de terres rares. Dans ce cas, l'élément dopant est alors lié de façon irreversible aux sesquisulfures, en ce sens que, par exemple, même des lavages très poussés de ces derniers ne permettent pas de l'éliminer. De tels lavages peuvent conduire par contre à l'élimination des éventuels sulfures et/ou polysulfures d'alcalin(s) ou d'alcalino-terreux présents à la surface des supports (avant enrobage), et donc non liés de façon irreversible à ces derniers.

Sans vouloir limiter la présente invention à une théorie, on peut ici avancer l'explication probable suivante:

On sait que les sesquisulfures de terres rares M₂S₃ cristallisent selon une structure cristallographique de type Th₃P₄, qui présente des lacunes au niveau du réseau des cations; cette structure lacunaire peut être symbolisée en donnant aux sesquisulfures la formule M_{10,66} [ ]_{1,33} S₁₆ (voir notamment à ce sujet, W.H. ZACHARIASEN, "Crystal Chemical Studies of the 5f-Series of Elements. The Ce₂S₃-Ce₃S₄ Type of Structure", Acta Cryst. , (1949), 2, 57).

Or, selon l'invention, des éléments alcalins et/ou alcalino-terreux peuvent être introduits dans ces lacunes cationiques, jusqu'à saturation ou non de ces dernières. La présence de l'élément dopant au sein des supports mis en oeuvre dans le cadre de l'invention peut être mise en évidence par simple analyse chimique. Par ailleurs, les analyses en diffraction X montrent qu'il y a conservation de la phase cristalline en Th₃P₄ du sesquisulfure, avec dans certains cas, une modification plus ou moins importante des paramètres de maille, fonction à la fois de la nature et de la quantité de l'élément dopant introduit.

De façon tout à fait inattendue et surprenante, il s'avère que cette insertion au sein du réseau cristallin du sesquisulfure confère à ce dernier des caractéristiques chromatiques nettement améliorées par rapport à tous les sesquisulfures de terres rares connus à ce jour. En outre, la présence de cet élément dopant peut avoir pour effet bénéfique de stabiliser aux hautes températures la structure cristalline du sesquisulfure considéré, et donc de conserver la couleur désirée dans une plus grande plage de température.

Selon un mode de réalisation préféré des compositions selon l'invention, I'élément dopant présent dans le support est un élément alcalin et est par exemple choisi, seul ou en mélange, parmi le lithium, le sodium et le potassium.

De manière encore plus préférentielle, l'alcalin est le sodium.

Parmi les éléments alcalino-terreux utilisables selon l'invention, on peut plus particulièrement citer le magnésium, le calcium, le baryum et le strontium.

Selon une autre caractéristique préférée desdites compositions, la quantité molaire en alcalin(s) et/ou alcalino-terreux présent(s) dans le support est au moins égale à 0,1%, et avantageusement comprise entre 5% et 50%, de la quantité molaire en la ou les terres rares présentes dans ce support.

La nature de la ou des terres rares, ainsi que le type de réseau cristallin du sesquisulfure, sont choisis en fonction de la couleur que l'on désire obtenir pour la composition ou le pigment. Dans tous les cas, on observe que, pour un élément dopant convenablement sélectionné, les compositions selon l'invention présentent alors des colorations beaucoup plus intenses que celles des sesquisulfures correspondants non dopés par un élément alcalin et/ou alcalino-terreux; par sesquisulfure correspondant, on entend le sesquisulfure contenant la ou les mêmes terres rares et présentant la même forme cristallographique.

Pour un sesquisulfure de terre rare donné, et donc de coloration donnée, l'invention permet ainsi d'accéder, après de simples essais de routine, à toute une gamme de compositions de couleurs améliorées, et ceci en jouant simplement sur la nature et/ou sur la concentration en élément dopant dans le support.

On donne ci-dessous des exemples de couleurs auxquelles il est possible d'aboutir avec les compositions selon l'invention, et ceci en fonction de la nature du support mis en jeu, cette liste n'étant bien entendu donnée qu'à titre purement illustratif et donc nullement limitatif:
- les compositions dont le support est à base de sulfures de cérium ont une couleur variant du brun au rouge selon les conditions de préparation, en particulier la température de calcination. Ils sont bruns ou rouges sang selon que l'on ait la phase Ce₂S₃ b orthorhombique (J.C.P.D.S. 20 269) ou la phase Ce₂S₃ γ cubique (J.C.P.D.S. 27 104)
- avec le lanthane, on obtient des compositions jaunes, et ceci avec une structure La₂S₃ cubique (J.C.P.D.S. 25 1041)
- une coloration verte peut être obtenue avec le néodyme, et une coloration vert-jaune avec le praséodyme. Les supports présentent alors respectivement la structure Nd₂S₃ cubique (J.C.P.D.S. 26 1450) et la structure Pr₂S₃ cubique (J.C.P.D.S. 27 481)
- on dispose d'un pigment jaune-marron avec le dysprosium de structure Dy₂S₃ cubique (J.C.P.D.S. 26 594)
- des compositions présentant différentes nuances de marron peuvent aussi être obtenus : ocre avec le terbium de structure Tb₂S₃ cubique, brun avec l'erbium de structure Er₂S₃ monoclinique (J.C.P.D.S. 21 324) et beige foncé avec l'yttrium de structure Y₂S₃ monoclinique (J.C.P.D.S. 22 996)
- d'autres exemples de couleurs que l'on peut obtenir sont enfin : brun-gris avec le samarium de structure Sm₂S₃ cubique (J.C.P.D.S. 26 1480), brun-vert avec le gadolinium de structure Gd₂S₃ γ cubique (J.C.P.D.S. 26 1424), vert-or avec le thullium de structure Tm₂S₃ monoclinique (J.C.P.D.S. 30 1364).

La coloration intrinsèque tant des supports-pigments que des compositions selon l'invention, peut par ailleurs être quantifiée au moyen des coordonnées chromatiques L*, a* et b* données dans le système CIE 1976 (L*,a*,b*) tel que défini par la Commission Internationale d'Eclairage et répertorié dans le Recueil des Normes Françaises (AFNOR), couleur colorimétrique n° X08-12 (1983). Elles sont déterminées au moyen d'un colorimètre commercialisé par la Société Pacific Scientific. La nature de l'illuminant est D65. La surface d'observation est une pastille circulaire de 12,5 cm² de surface. Les conditions d'observation correspondent à une vision sous un angle d'ouverture de 10°. Dans les mesures données, la composante spéculaire est exclue.

L* donne une mesure de la réflectance (nuance clair/sombre) et varie ainsi de 100 (blanc) à 0 (noir).
a* et b* sont les valeurs des tendances colorées:
a* positif = rouge
a* négatif = vert
b* positif = jaune
b* négatif = bleu
L* représente donc la variation du noir au blanc, a* la variation du vert au rouge et b* la variation du bleu au jaune.

Ainsi, selon la présente invention, et à titre seulement d'exemple, quand la terre rare est le cérium et que le sesquisulfure est sous sa forme cristallographique γ cubique, on dispose de compositions ou pigments présentant les coordonnées trichromatiques tout à fait remarquables suivantes:
- L* au moins égal à 30, et notamment comprise entre 30 et 60,
- a* au moins égal à 30, et notamment comprise entre 35 et 65,
- b* compris généralement entre 0 et 40.

De telles coordonnées, et en particulier la coordonnée chromatique a*, correspondent à une couleur rouge intense tout à fait exceptionnelle pour un sulfure de cérium Ce₂S₃ γ cubique, et est équivalente voire supérieure à celle des pigments rouges de référence, à savoir le sélénure de cadmium et le sulfosélénure de cadmium. En outre, l'un des avantages d'un tel pigment est qu'il ne présente pas les problèmes de toxicité liés à la présence de métaux lourds, comme cela est généralement le cas dans les pigments de l'art antérieur.

Comme indiqué précédemment, la présente invention a également pour objet un procédé de fabrication industriel permettant d'accéder aux nouvelles compositions selon l'invention. Toutefois, avant d'exposer ce procédé plus précisément, on commencera tout d'abord par donner ci-dessous les détails d'un procédé particulièrement avantageux convenant à la synthèse des sesquisulfures dopés par des alcalins et/ou des alcalino-terreux tels que décrits ci-avant et qui sont utilisés ici à titre de supports, ces derniers constituant finalement l'une des matières premières essentielles nécessaire à la préparation des compositions selon l'invention. On peut d'ores et déja noter que le procédé qui suit convient particulièrement bien, entre autres, pour accéder à des compositions de support dans lesquelles le sesquisulfure de terres rares se présente sous une forme cristalline cubique, et notamment γ cubique.

Ce procédé consiste à réaliser un mélange initial contenant au moins un composé de terre rare, du soufre et au moins un composé d'un élément alcalin et/ou alcalino-terreux (élément dopant), à chauffer ledit mélange initial jusqu'à l'obtention de la phase sesquisulfure désirée, et ceci sous une atmosphère non oxydante, de préférence réductrice, puis à refroidir le mélange ainsi traité.

Selon une variante avantageuse de ce procédé, le chauffage du mélange initial est réalisé en présence d'un agent réducteur. La quantité d'agent réducteur ajoutée est alors déterminée de manière à maintenir une pression partielle d'oxygène très basse dans le réacteur. Ainsi, la quantité d'agent réducteur mise en oeuvre est avantageusement suffisante pour consommer l'oxygène libre et/ou combiné contenu dans le mélange initial.

Dans un premier mode possible de réalisation de cette variante, un agent réducteur est ajouté directement au mélange initial. Cet agent est généralement à base de carbone, tel que, par exemple, le graphite, le coke, la lignite ou bien encore un composé organique générant du carbone par chauffage. Ce peut être aussi un réducteur métallique, par exemple l'aluminium.

Selon un second mode possible de réalisation de cette variante, l'agent réducteur est contenu dans le gaz formant l'atmosphère non oxydante. On réalise alors avantageusement un balayage du mélange initial avec un gaz non oxydant, de préférence un gaz inerte, contenant un agent réducteur tel que par exemple l'hydrogène ou le monoxyde de carbone CO. On peut ainsi mettre en oeuvre un mélange d'hydrogène avec un gaz inerte, tel qu'un mélange argon/hydrogène ou azote/hydrogène, ou bien encore un mélange argon/CO ou azote/CO. Ce balayage peut également être réalisé par de l'hydrogène ou du monoxyde de carbone seuls.

Avantageusement, lors de la montée en température, on maintient le mélange à une température intermédiaire, notamment comprise entre 250°C et 500°C, avant de porter celui-ci à la température correspondant à la formation du sesquisulfure désiré. Ce maintien à une température intermédiaire est réalisé pendant une durée comprise généralement entre 15 minutes et 1 heure.

Les composés de terres rares convenables pour la mise en oeuvre du procédé ci-dessus sont par exemple choisis dans le groupe constitué par les composés oxycarbonés de terres rares, les sulfates, les oxydes de terres rares. A titre de composés oxycarbonés de terres rares, on peut citer notamment les carbonates, oxalates, acétates, malonates, tartrates de terres rares.

Concernant les composés d'alcalins ou d'alcalino-terreux utilisables dans ce même procédé, on peut citer par exemple les oxydes, les sulfures ou polysulfures, les sulfates, les composés oxycarbonés tels qu'oxalates, carbonates ou acétates d'alcalins ou d'alcalino-terreux. De préférence, on met en oeuvre des carbonates de ces éléments.

La quantité d'élément alcalin ou alcalino-terreux ajoutée est déterminée pour avoir un rapport molaire : élément dopant / terre(s) rare(s) compris généralement entre 0,05 et 0,5, et de préférence entre 0,15 et 0,30 dans le mélange initial.

Par ailleurs, la quantité de soufre présent dans le mélange initial est déterminée pour avoir un rapport molaire : soufre / terre(s) rare(s) supérieur ou égal à 1,5 et de préférence supérieur à 2.
Le soufre peut être introduit sous forme libre (soufre élémentaire solide ou gazeux) ou sous forme d'un composé soufré précurseur, par exemple Na₂S ou CS₂.

De préférence, on utilise du soufre élémentaire à l'état solide.

Le mélange initial peut bien entendu comprendre plusieurs composés de terres rares et/ou d'alcalins et/ou d'alcalino-terreux, comme souligné ci-avant.

Le mélange initial ainsi obtenu est ensuite chauffé à une température et pendant un temps suffisants pour obtenir la phase sesquisulfure désirée, ce temps étant généralement d'autant plus court que la température est élevée.

Cette température dépend bien entendu du sesquisulfure considéré.

Avantageusement, le mélange est chauffé à une température supérieure à 900°C, généralement comprise entre 1000°C et 1400°C, de préférence 1150-1300°C, et ceci pendant au moins 30 minutes, de préférence entre 30 minutes et deux heures.

Le produit ainsi obtenu peut ensuite éventuellement être soumis à un ou des lavages, par exemple à l'eau, pour diminuer la teneur en alcalin(s) et/ou alcalino-terreux non liés.

Si nécessaire, le produit obtenu peut enfin être broyé (broyage à jet d'air ou autre) pour obtenir un diamètre moyen de grains compris entre 0,2 µm et 5 µm.Toutefois, selon le procédé tel qu'exposé ci-dessus, on arrive généralement à cette granulométrie sans avoir à broyer le produit, ce qui constitue un avantage très important d'un point de vue économique.

Le produit obtenu présente alors une très bonne pureté phasique (absence d'oxysulfure notamment) et des cooordonnées chromatiques remarquablement élevées dans la couleur spécifique du sesquisulfure de terre rare considéré.

Comme souligné précédemment, ces produits (sesquisulfures de terres rares dopés) sont destinés à constituer le support des compositions colorés selon la présente invention.

On revient maintenant à l'exposé du procédé de préparation de ces compositions de type core-shell.

Comme indiqué ci-avant, ce procédé repose essentiellement sur la précipitation d'un oxyde transparent formant une couche protectrice à la surface d'un support tel que décrit ci-dessus. Le principe de préparation des nouvelles compositions selon l'invention consiste donc essentiellement à mettre en présence la particule ou support minéral à traiter et un précurseur de l'oxyde formant couche, et à précipiter cet oxyde.

Il importe de noter ici que l'invention n'est pas limitée à un procédé particulier de précipitation. Aussi, la description du ou des procédés qui va être faite ci-dessous est donnée à titre illustratif et ne doit pas être considérée comme limitative. On notera en outre qu'il existe une éxigence générale commune à respecter pour la bonne marche de tous ces procédés, à savoir qu'il convient dans toute la mesure du possible d'éviter que les sesquisulfures de terres rares utilisés à titre de support ne soient mis en contact, avant ou pendant l'étape de précipitation, avec un milieu trop acide qui pourrait provoquer la décomposition de ces derniers.

Ce procédé va tout d'abord être décrit dans le cas particulier et préféré où l'oxyde formant couche est la silice.

Ce procédé peut être mis en oeuvre essentiellement selon deux variantes.

Dans le cas de la première variante, et qui est ici préférée à la deuxième variante exposée ci-après, le procédé consiste essentiellement à préparer la silice par hydrolyse d'un alkyl-silicate.

Plus précisément, on met en présence le support et un alkyl-silicate, on hydrolyse cet alkyl-silicate et on sépare le pigment formé et la phase liquide du milieu réactionnel.

On notera que le principe de cette voie est plus particulièrement décrit dans l'article de STÖBER et al., Journal of Colloïd and Interface Science, 26, pp. 62-69 (1968) et dont l'enseignement est incorporé à la présente description.

Généralement, il est préférable d'effectuer l'hydrolyse en présence d'une base qui joue alors le rôle de catalyseur.

On procède en formant un milieu réactionnel par mélange d'eau, d'alcool, du support qui est alors mis en suspension, et éventuellement d'une base, et en introduisant ensuite l'alkyl-silicate. La réaction est de préférence conduite sous agitation.

On peut utiliser comme base l'ammoniaque. Les alcools mis en oeuvre sont généralement des alcools aliphatiques, tel que par exemple du butanol. L'alkylsilicate est de préférence introduit avec un alcool.

Bien que la réaction puisse être conduite à température ambiante, on note toutefois une amélioration sensible de la qualité de la couche d'enrobage lorsque la température d'hydrolyse est choisie supérieure à cette température ambiante, en particulier comprise entre 40°C et 70°C.

Il est aussi possible de faire un pied de cuve à base d'alcool et d'alkylsilicate, et d'y introduire ensuite l'eau ou un mélange eau-base.

Comme alkyl-silicate, on peut utiliser en particulier le silicate d'éthyle.

Après réaction et précipitation, le pigment obtenu est séparé du milieu réactionnel par toute technique connue en soi, en particulier par centrifugation ou par filtration, et est ensuite généralement lavé par de l'alcool, puis enfin séché.

Concernant la deuxième variante, le procédé consiste à mettre en présence, à mélanger et à faire réagir le support, un silicate et un acide, ce par quoi on précipite de la silice.

Selon un premier mode de réalisation de cette deuxième variante, l'acide et le silicate sont ajoutés simultanément dans le milieu réactionnel. Plus précisément, on forme tout d'abord une suspension aqueuse du support, et ensuite on ajoute simultanément l'acide et une solution aqueuse de silicate dans le milieu réactionnel.

Selon un mode de réalisation particulier, on peut alors introduire l'acide et le silicate en maintenant constant le pH du milieu réactionnel. Généralement, ce pH est fixé à une valeur comprise entre 8 et 9.

La température de réaction peut varier largement. En général, cette température est comprise entre 60 et 100°C.

Un deuxième mode (préféré au premier ci-dessus) de réalisation de cette deuxième variante du procédé selon l'invention consiste à former tout d'abord une suspension du support dans une solution aqueuse de silicate, et à ensuite introduire l'acide dans la suspension ainsi formée. Dans ce cas, le pH du milieu de précipitation est avantageusement fixé à une valeur supérieure à 7, et de préférence supérieure à 8, et ceci de manière à éviter une dissolution néfaste du support à base de sulfures de terres rares.

Dans cette deuxième variante, les conditions de température sont identiques à celles décrites dans la première.

En ce qui concerne le silicate, on utilise généralement un silicate alcalin et plus particulièrement les silicates de sodium, de potassium ou de lithium.

Pour les acides, on utilise habituellement un acide choisi dans le groupe comprenant les acides sulfurique, nitrique, chlorhydrique, carbonique. L'acide est généralement employé sous la forme d'une solution aqueuse.

La séparation entre le pigment d'une part et la phase liquide du milieu réactionnel d'autre part obtenus selon les procédés décrits ci-dessus se fait ensuite d'une manière connue en soi, par exemple par filtration. Le pigment séparé est ensuite séché.

Dans le cas où l'oxyde formant couche est l'alumine, plusieurs variantes peuvent aussi être envisagées.

Selon une première variante, on met en présence et on fait réagir le support, un aluminate et un acide, ce par quoi on précipite de l'alumine.

Selon un premier mode de réalisation de cette première variante, on pourra introduire simultanément l'aluminate et l'acide dans une suspension aqueuse du support. Dans ce cas, on peut opérer de manière à maintenir constant le pH du milieu réactionnel, ce pH étant de préférence supérieur à 7, et encore plus préférentiellement supérieur à 8.

Selon un deuxième mode de réalisation de cette première variante, on forme tout d'abord une suspension du support dans une solution d'aluminate, puis on introduit l'acide dans cette suspension. Là encore, on veillera à ce que le pH du milieu de réaction lors de l'introduction de l'acide reste de préférence supérieur à 7, ou même supérieur à 8.

D'une manière générale dans cette première variante, on utilise un aluminate alcalin. L'acide peut être, quant à lui, par exemple de l'acide chlorydrique ou de l'acide nitrique.

La seconde variante de préparation d'un pigment composite selon l'invention avec l'alumine comme oxyde formant couche consiste à mettre en présence, à mélanger et à faire réagir, le support, un sel d'aluminium et une base, ce par quoi l'on précipite de l'alumine.

Ainsi, on pourra par exemple partir d'un pied de cuve constitué par une suspension aqueuse du support, puis introduire de manière simultanée la base et le sel d'aluminium dans cette suspension.

La base utilisée est généralement la soude ou l'ammoniaque, et le sel d'aluminium pouvant être par exemple un halogénure d'aluminium tel que le chlorure d'aluminium, ou bien encore le nitrate d'aluminium.

Enfin, selon une troisième variante, on peut préparer les compositions avec l'alumine formant couche en obtenant ladite alumine par hydrolyse d'un alcoolate d'aluminium.

Cette variante est à rapprocher de celle décrite plus haut pour l'hydrolyse d'un alkyl-silicate.

On procède alors en mettant en présence le support et un alcoolate d'aluminium, on hydrolyse cet alcoolate et on sépare le pigment formé et la phase liquide du milieu réactionnel.

Tout ce qui a été décrit plus haut dans le cadre de l'hydrolyse d'un alkylsilicate s'applique ici par analogie, notamment en ce qui concerne l'emploi d'une base et le mode d'introduction des réactifs.

On peut utiliser à titre d'alcoolates d'aluminium le méthylate, I'éthylate, l'isopropylate, le butylate d'aluminium, ces alcoolates étant sous forme liquide ou solide en dispersion ou en solution dans un solvant organique, par exemple le benzène ou l'alcool correspondant.

Dans le cas de la préparation d'un pigment composite comprenant le dioxyde de titane comme oxyde formant couche, on peut envisager différentes méthodes.

La première consiste à réaliser une précipitation de TiO2 en introduisant dans une suspension aqueuse du support un sel de titane d'une part tel que TiCl4, TiOCl2 ou TiOSO4, et une base d'autre part telle que de la soude ou de l'ammoniaque, l'introduction du sel et de la base se faisant de manière simultanée. On peut ensuite procéder à un mûrrissement du pigment.

Une deuxième méthode consiste à hydrolyser un titanate d'alkyle, par exemple le titanate d'isopropyle. Cette méthode est du même type que celle qui a été décrite plus haut pour l'hydrolyse d'un alkyl-silicate. On procède donc en mettant en présence le support et un titanate d'alkyle, puis on hydrolyse le titanate d'alkyle et on sépare le pigment formé et la phase liquide du milieu. Tout ce qui a été décrit plus haut à ce sujet concernant le mode d'introduction des réactifs s'applique ici.

Dans le cadre de la préparation de compositions comprenant l'oxyde de zirconium comme oxyde formant couche, les méthodes de préparation sont du même type que celles qui viennent d'être définies plus haut pour le dioxyde de titane, à savoir précipitation par réaction entre un sel de zirconium et une base ou hydrolyse d'un zirconate d'alkyle.

De même, dans le cadre de la préparation de compositions comprenant un ou des oxydes de terres rares à titre d'oxydes formant couche, on peut procéder soit par hydrolyse d'un alcoolate de terre rare, soit par précipitation en introduisant, et ceci de manière simultanée, dans une suspension aqueuse du support d'une part un sel de terre rare (nitrate ou chlorure) et une base d'autre part (soude, ammoniaque, par exemple).

Enfin, dans le cas du zircon formant couche autour du support, il est possible de procéder de la manière suivante : on co-hydrolyse dans une suspension aqueuse du support un alcoolate de zirconium d'une part et un alcoolate de silicium d'autre part, et ceci en présence de fluorure de sodium NaF, on récupère le pigment ainsi formé puis on cacine ce pigment de manière à transformer la couche d'enrobage précipitée en une phase de zircon, NaF jouant alors un rôle de fondant aidant à réaliser ladite transformation à la température la plus basse possible.

D'une manière générale, on notera qu'il est toujours possible de rester dans le cadre de la présente invention en préparant des compositions ayant soit plusieurs oxydes formant plusieurs couches succéssives, soit des mélanges d'oxydes ou oxydes mixtes formant une même couche.

En outre, on peut également mentioner la possibilité d'effectuer un ou des prétraitements du support (avant enrobage) dans le but d'améliorer encore certaines de ses caractéristiques ou propriétés au cours du traitement d'enrobage, ou même d'améliorer certaines des caractéristiques ou propriétés du pigment composite résultant.

La granulométrie des compositions récupérés est fine et régulière, et peut se situer entre 1 et 5 microns. Avec une telle granulométrie, les produits sont directement utilisables comme pigments, notamment pour matières plastiques. Comme indiqué précédemment dans la description, cette granulométrie est en relation directe avec la granulométrie initiale des particules de support mises en oeuvre dans le procédé d'enrobage selon l'invention. Cette dernière doit donc être choisie en fonction et en prévision de la granulométrie désirée pour le pigment composite final en vue d'une application particulière donnée.

Outre leurs excellentes couleurs intrinsèques et leur stabilité chimique et/ou thermique améliorée, les compositions selon l'invention possèdent un très bon pouvoir de coloration et un très bon pouvoir opacifiant, et , de ce fait, elles conviennent parfaitement à la coloration de nombreux matériaux, tels que plastiques, peintures et autres et elles peuvent ainsi constituer des pigments colorés ou entrer dans la composition de pigments colorés. A cet égard, la polyvalence des compositions selon l'invention constitue l'un de leurs grands atouts.

Ainsi, et plus précisémment encore, les compositions ou les pigments de l'invention peuvent être utilisés dans la coloration de matières plastiques qui peuvent être du type thermoplastiques ou thermodurcissables.

Comme résines thermoplastiques susceptibles d'être colorées selon l'invention, on peut citer, à titre purement illustratif, le chlorure de polyvinyle, l'alcool polyvinylique, le polystyrène, les copolymères styrène-butadiène, styrèneacrylonitrile, acrylon itrile-butadiène-styrène (A.B.S.), les polymères acryliques notamment le polyméthacrylate de méthyle, les polyoléfines telles que le polyéthylène, le polypropylène, le polybutène, le polyméthylpentène, les dérivés cellulosiques tels que par exemple l'acétate de cellulose, I'acéto-butyrate de cellulose, l'éthylcéllulose, les polyamides dont le polyamide 6-6.

Concernant les résines thermodurcissables pour lesquelles les compositions ou pigments selon l'invention conviennent également, on peut citer, par exemple, les phénoplastes, les aminoplastes notamment les copolymères urée-formol, mélamine-formol, les résines époxy et les polyesters thermodurcissables.

On peut également mettre en oeuvre les compositions ou pigments de l'invention dans des polymères spéciaux tels que des polymères fluorés en particulier le polytétrafluoréthylène (P.T.F.E.), les polycarbonates, les élastomères silicones, les polyimides.

Dans cette application spécifique pour la coloration des plastiques, on peut mettre en oeuvre les compositions ou pigments de l'invention directement sous forme de poudres. On peut également, de préférence, les mettre en oeuvre sous une forme pré-dispersée, par exemple en prémélange avec une partie de la résine, sous forme d'un concentré pâte ou d'un liquide ce qui permet de les introduire à n'importe quel stade de la fabrication de la résine. Ce dernier point constitue un avantage particulièrement important des compositions selon l'invention.

Ainsi, les produits selon l'invention peuvent être incorporés dans des matières plastiques telles que celles mentionnées ci-avant dans une proportion pondérale allant généralement soit de 0,01 à 5% (ramenée au produit final) soit de 40 à 70% dans le cas d'un concentré.

Les produits de l'invention peuvent être également utilisés dans le domaine des peintures et lasures et plus particulièrement dans les résines suivantes : résines alkydes dont la plus courante est dénommée glycérophtalique; les résines modifiées à l'huile longue ou courte; les résines acryliques dérivées des esters de l'acide acrylique (méthylique ou éthylique) et méthacrylique éventuellement copolymérisés avec l'acrylate d'éthyle, d'éthyl-2 hexyle ou de butyle; les résines vinyliques comme par exemple l'acétate de polyvinyle, le chlorure de polyvinyle, le butyralpolyvinylique, le formalpolyvinylique, et les copolymères chlorure de vinyle et acétate de vinyle ou chlorure de vinylidène; les résines aminoplastes ou phénoliques le plus souvent modifiées; les résines polyesters; les résines polyuréthanes; les résines époxy; les résines silicones.

Généralement, les compositions ou pigments sont mis en oeuvre à raison de 5 à 30% en poids de la peinture, et de 0,1 à 5% en poids du lasure.

Enfin, les produits selon l'invention sont également susceptibles de convenir pour des applications dans l'industrie du caoutchouc, notamment dans les revêtements pour sols, dans l'industrie du papier et des encres d'imprimerie, dans le domaine de la cosmétique, ainsi que nombreuses autres utilisations comme par exemple, et non limitativement, le finissage des cuirs et les revêtements stratifiés pour cuisines et autres plans de travail, les céramiques.

En ce qui concerne plus particulièrement la cosmétique, les compositions ou pigments de l'invention peuvent être utilisés dans les vernis à ongles et dans les fards tels que rouges à lèvres, fards secs, fards gras ou fonds de teint.

lls peuvent donc être mises en oeuvre dans les vernis à ongles qui contiennent généralement :
- un agent filmogène à bas de nitrocellulose,
- une résine, résine dammer naturelle ou résine synthétique du type formaldéhyde-sulfamide, résine polystyrène, résine polyvinylique etc...,
- un plastifiant par exemple, le phtalate de diéthyle, le phtalate de dibutyle, le phtalate de dioctyle, le tricrésylphosphate, le stéarate de n-butyle, le diacétate de résorcine ou leur mélange,
- un dissolvant tel que l'alcool éthylique, isopropylique, butylique, isobutylique, I'acétate d'éthyle, I'acétate de butyle ou le plus souvent le mélange de ces solvants,
- un diluant, notamment le toluène ou le xylène,
- éventuellement d'autres additifs, parfum ou produit nacrant (flocons de mica enrobés d'oxychlorure de bismuth ou de bioxyde de titane).

On donne ci-après un exemple de composition-type :
- de 10 à 15% en poids de nitrocellulose,
- de 10 à 15% en poids de résine,
- de 3 à 5% en poids de plastifiant(s),
- de 3 à 5% en poids de pigment(s),
- q.s.p. 100% en poids de solvant(s).

Généralement, les compositions ou pigments sont broyés dans une masse plastique constituée de nitrocellulose et de plastifiant(s) qui est ensuite mise en solution dans le(s) solvant(s).

Une autre application des compositions ou pigments de l'invention est celle des rouges à lèvres.

Les compositions ou pigments sont mis en oeuvre le plus souvent à raison d'une concentration pondérale de 5 à 15% exprimée par rapport à la formulation totale qui renferme :
- un excipient formé d'un mélange de divers corps pour assurer la consistance : cire d'abeille, cire de carnauba, ozocérites, paraffine, cires synthétiques ou leur mélange et d'un excipient mou permettant d'ajuster la consistance tel que le beurre de cacao, la vaseline, les huiles blanches hydrogénées par exemple, l'huile de palme, d'arachide ou de ricin,
- divers additifs notamment un parfum ou arome et le myristate d'isopropyle ou le palmitate d'isopropyle qui donne du glissant,
- un solvant intermédiaire pour mettre en suspension le pigment dans la phase lipophyle qui peut être l'huile de ricin ou un glycol comme le polyoxyéthylèneglycol 400 ou des esters d'acides gras : monoricinoléate de propylène glycol, myristate d'isopropyle, palmitate d'isopropyle, stéarate de butyle.

Les fards à yeux et les fards à joues peuvent se présenter sous forme de fards secs ou de fards gras. La teneur en compositions ou pigments dans de tels fards peut varier dans de larges limites de 5 à 20%.

Les fards secs sont des poudres (talc, carbonate de magnésium, stéarate de zinc) qui sont chargées en pigments et agglomérées soit avec de la méthylcellulose, soit avec des stéarates.

On donne, à titre d'exemple la composition d'un fard à paupières :
- silicate d'aluminium et de magnésium (Veegum F): 7% en poids
- talc : 50% en poids
- oxyde de zinc : 4% en poids
- stéarate de zinc : 11% en poids
- kaolin : 10% en poids
- pigment : 18% en poids

Les compositions ou pigments de l'invention peuvent également être employés dans les formulations de fonds de teint.

Les fonds de teint se présentent sous forme d'émulsion en général du type huile dans l'eau.

La phase lipophyle comprend le plus souvent :
- un composant huileux tel que l'huile de vaseline, des esters d'acides gras et d'alcools éventuellement gras, par exemple, l'oléate d'oléyle, l'oléate de décyle, le stéarate d'octyle, l'adipate de di-n-butyle, le myristate d'isopropyle, le palitate d'isopropyle, le stéarate d'isopropyle, les esters d'acides caprique et caprylique d'alcools gras saturés ayant de 12 à 18 atomes de carbone, une huile de silicone ou leur mélange entre eux,
- un agent émulsifiant du type anionique et/ou non-ionique et plus précisément les sels d'acides gras, stéarate de sodium, de potassium ou d'ammonium, palmitate de sodium : les esters de sorbitan et d'acides gras tels que, par exemple, l'acide laurique, l'acide palmitique, l'acide stéarique; les esters polyoxyéthylénés de sorbitan et d'acides gras contenant de 4 à 20 moles d'oxyde d'éthylène par mole d'ester : les alcools gras polyoxyéthylénés contenant de 2 à 23 moles d'oxyde d'éthylène par mole d'alcool, ledit alcool pouvant être notamment l'alcool laurique, l'alcool cétylique, l'alcool stéarylique, l'alcool oléylique; le mono-et distéarate de glycérol, le mono-et dioléate de glycérol; les acides gras poxyloxyéthylénés et en particulier le stéarate polyoxyéthyléné contenant de 18 à 100 moles d'oxyde d'éthylène par mole d'acide,
- un agent permettant d'ajuster la consistance qui peut être un alcool gras ou un acide gras et plus précisément l'alcool cétylique, l'alcool stéarylique, l'acide stéarique.

Quant à la phase hydrophile, elle est constituée d'eau, de préférence distillée et de divers additifs, notamment :
- un agent humectant qui peut être, par exemple, le propylèneglycol, le glycérol, le sorbitol,
- un agent conservateur et plus particulièrement l'ophénylphénol et les acides suivants, leurs sels (Na, K, NH₄) ou leurs esters ayant de 1 à 4 atomes de carbone : l'acide benzoïque, l'acide salicylique, l'acide sorbique, l'acide p-hydroxybenzoïque,
- un agent de stabilité notamment les dérivés cellulosiques dont la carboxyméthylcellulose et la gomme Xanthane.

Une illustration d'une formulation pour fonds de teint est donnée ci-après :--
- phase lipophyle :
   - huile de vaseline : 15% en poids
   - mono-et distéarate de glycérol : 4% en poids
   - alcool cétylique : 1% en poids
- phase hydrophyle :
   - eau distillée q.s.p. : 100% en poids
   - propylène glycol : 3% en poids
   - para-oxybenzoate de méthyle : 0,05% en poids
   - para-oxybenzoate de propyle : 0,1% en poids
- pigment coloré : 1 à 10% en poids
- oxyde de titane : 3% en poids

La préparation des formulations de fonds de teint est conduite en dispersant d'abord le pigment dans la phase lipophile maintenue vers 60-80°C puis la phase hydrophyle maintenue à une des températures comprises dans l'intervalle précité est ajoutée sous agitation et lentement dans la phase lipophile.

Dans l'exposé qui précède, il est donné des exemples de formulations destinées à la cosmétique dans lesquelles peuvent convenir les compositions ou pigments de l'invention et il va sans dire que ces exemples de même que les différents composants cités ne présentent aucun caractère limitatif et ne sont donnés qu'à titre illustratif.

Des exemples illustrant l'invention vont maintenant être donnés.

Ces exemples illustrent tant la synthèse et la caractérisation de certains des supports et des compositions résultants conformes à l'invention, que l'utilisation de ces dernieres pour une de leurs applications possibles dans le domaine de la coloration des matières plastiques et de la cosmétique.

### Exemple 1

On réalise ici la préparation d'un sesquisulfure de cérium Ce₂S₃ γ cubique, dopé au sodium.

Dans un mortier, on introduit 58 g d'oxalate de cérium Ce₂(C₂O₄)₃,2H₂O; 48 g de soufre élémentaire; et 2,2 g de carbonate de sodium anhydre; le rapport molaire initial Na/Ce dans le mélange est alors de 0,20.

L'ensemble est alors broyé de façon à obtenir un mélange bien homogène.

Ce mélange est ensuite placé dans une nacelle en carbone, laquelle est introduite dans un four tubulaire étanche à l'air. On purge ce four, puis on y réalise un bayage continu d'argon chargé à 10% d'hydrogène.

La température du four est portée à 325 °C, à raison de 5°C/mn, avec un palier d'une heure à cette température.

Puis la température est portée à 1100°C, toujours à raison de 5°C/mn, avec à nouveau un palier de quatre à cette température.

On revient ensuite à la température ambiante, le refroidissement se faisant à raison de 5°C/mn

Le produit récupéré est ensuite lavé une fois avec de l'eau permutée.

Le produit obtenu présente alors une couleur rouge très intense.

L'analyse en diffraction X montre que l'on obtient la seule phase Ce₂S₃ γ cubique, avec un paramètre de maille égal 8,637 A.

La teneur en sodium du produit est 2,25 % en poids (Na/Ce₂S₃).

Ses coordonnées chromatiques sont les suivantes :
L* = 48
a* = 47
b* = 36

Après simple désagglomération (broyage peu poussé, type jet d'air) du produit, on obtient une poudre présentant une granulométrie moyenne (f₅₀) de 2,5 µm (mesure par granulomètre laser CILAS).

On procède ensuite sur ce produit à deux tests, l'un de stabilité thermique, l'autre de stabilité chimique en milieu acide.

La stabilité thermique a été évaluée au moyen d'un test basé sur la détermination de la température de démarrage de reprise de poids en analyse ATG (montée en température de 6°C/mn et sous balayage d'air).

Le test de stabilité chimique est effectuée dans une solution d'acide chlorhydrique 0,5 N (acide de densité 1,18 dilué à 5% dans de l'eau).

Ainsi, par analyse de la courbe ATG, on observe que la dégradation de la stabilité thermique du produit débute à partir de 260°C.

De même, on observe que le produit se dissout instantannément dans la solution d'acide chlorhydrique ci-dessus définie.

### Exemple 2

On réalise ici la préparation d'un pigment composite Ce₂S₃(coeur)/SiO₂ (enveloppe) à partir du sulfure de cérium obtenu à l'exemple 1.

Les quantités et la nature des réactifs mis en jeu sont les suivantes (par Kg de mélange total) :
- Sulfure de cérium dopé sodium : 53,29 g/Kg
- Solution d'ammoniaque à 33% : 114,60 g/Kg
- Tétraéthylorthosilicate : 44,34 g/Kg
- Butanol : 787,77 g/Kg

Le sulfure de cérium dopé sodium est ajouté dans la solution hydroalcoolique ammoniacale puis dispersé dans cette dernière par ultrasonification. On ajoute ensuite, sous agitation, le silicate d'éthyle dans la suspension de particules de sulfure de cérium obtenue précédemment. Après deux heures de réaction, les nouvelles particules obtenues sont récupérées par centrifugation, puis lavées deux fois par de l'ethanol anhydre. Les particules lavées sont ensuite séchées à 50°C dans une étuve pendant 15 heures.

Les analyses en MEB confirment que l'on obtient des particules composites constituées d'un coeur de sulfure de cérium dopé sodium enrobé d'une couche de silice. Par ailleurs, les mobilités electrophorétiques de ces particules sont identiques à celles d'une silice pure.

La granulométrie moyenne (CILAS) de ces particules composites est de 5 µm (f₅₀).

Leurs coordonnées trichromatiques sont les suivantes:
- L* = 45,5
- a* = 49
- b* = 39

Selon le test de stabilité thermique défini ci-avant, on observe que la reprise en poids du produit n'apparait pas avant 410°C.

Enfin, il faut attendre 3 à 4 minutes pour dissoudre totalement ce produit dans la solution d'acide chlorhydrique 0,5 N.

Les deux derniers résultats ci-dessus dénotent une stabilité thermique d'une part et une stabilité chimique d'autre part nettement améliorées des compositions selon l'invention par rapport aux compositions correspondants non enrobés. On notera également l'amélioration sensible de la valeur de la coordonnée chromatique a*.

### Exemple 3 comparatif

On a reproduit ici l'exemple 1 de la demande de brevet EP-A- 0 203 838.

On a ainsi obtenu un sesquisulfure de cérium Ce2S3 γ cubique, de couleur rouge, présentant les coordonnées trichromatiques suivantes:
- L* = 35
- a* = 30
- b* = 14

Pour une couleur rouge, ces coordonnées sont nettement inférieures à celles du pigment dopé correspondant préparé à l'exemple 1, ainsi qu'à celles du pigment composite préparé à l'exemple 2.

En outre, ce produit se dissout instantannément dans la solution d'acide chlorhydrique 0,5 N.

Enfin, la dégradation thermique du produit, toujours selon le test défini ci-avant, est observée à 260°C.

### Exemple 4

On réalise ici la préparation d'un sesquisulfure de lanthane La2S3 γ cubique, dopé au potassium.

On prépare ainsi un mélange initial constitué de :
- 59 g d'oxalate de lanthane La₂(C₂O₄)₃,2H₂O
- 48 g de soufre élémentaire
- 4,24 g de carbonate de potassium anhydre.

On reproduit ensuite le mode opératoire de l'exemple 1, à cette différence près que l'étape de calcination finale se fait à 1200°C pendant une heure.

Le produit final obtenu présente une couleur jaune.

Son analyse par diffraction X montre que l'on obtient essentiellement la phase La₂S₃ g cubique, avec quelques traces de KLaS₂.

Sa teneur en potassium est de 4% en poids (K/La₂S₃).

La granulométrie moyenne du produit (f₅₀) est de 3 µm (CILAS).

Ses coordonnées chromatiques sont les suivantes :
- L* = 80
- a* = -3
- b* = 46

Selon le test défini ci-avant, la stabilité thermique du produit n'excède pas 270°C.

Par ailleurs, ce produit se dissout instantannément dans la solution d'acide chlorhydrique 0,5 N.

### Exemple 5

On réalise ici la préparation d'un pigment composite La₂S₃(coeur)/SiO₂(écorce) à partir du sulfure de lanthane obtenu à l'exemple 4.

Les quantités et la nature des réactifs mis en jeu sont les suivantes (par Kg de mélange total) :
- Sulfure de lanthane dopé potassium : 52,68 g/Kg
- Solution d'ammoniaque à 32% : 113,26 g/Kg
- Tétraéthylorthosilicate : 43,83 g/Kg
- Butanol : 779, 87 g/Kg

On procède ensuite selon le mode opératoire donné à l'exemple 2.

Les analyses en MEB et en MET confirment que l'on obtient des paticules composites constituées d'un coeur de sulfure de lanthane dopé au potassium enrobé d'une couche de silice. Par ailleurs, les mobilités electrophorétiques de ces particules sont identiques à celles d'une silice pure.

La granulométrie moyenne (f₅₀) de ces particules composites est de 5 µm (CILAS).

Leurs coordonnées trichromatiques sont identiques à celles du sulfure de lanthane préparé à l'exemple 4 et utilisé ici à titre de support.

La stabilité thermique du produit (telle qu'appréciée par le test défini ci-avant) est de l'ordre de 410°C.

Par ailleurs, il faut attendre 3 à 4 minutes pour dissoudre totalement ce produit dans la solution d'acide chlorhydrique 0,5N.

### Exemple 6 comparatif

On a reproduit ici l'exemple 3 de la demande de brevet EP-A- 0 203838. On a ainsi obtenu un sesquisulfure de lanthane La2S3 γ cubique, de couleur jaune, présentant les coordonnées trichromatiques suivantes :
- L* = 70
- a* = -5
- b* = 39

Pour une couleur jaune, ces coordonnées chromatiques sont nettement inférieures à celles obtenues pour le pigment dopé correspondant de l'exemple 4, ainsi qu'à celles du pigment composite de l'exemple 5.

En outre, ce produit se dissout instantannément dans la solution d'acide chlorhydrique 0,5 N.

Enfin, la dégradation thermique du produit, toujours selon le test défini ci-avant, est observée à 270°C.

### Exemple 7

Cet exemple a pour but d'illustrer l'aptitude des compositions selon l'invention pour la coloration des plastiques.

20 g du pigment composite rouge préparé à l'exemple 2 (coeur de Ce₂S₃ dopé sodium/écorce SiO₂; f₅₀ = 5 µm; L* =45,5 ; a* = 49 ; b* = 39) sont mélangés en cube tournant à 2 Kg d'un polypropylène de référence Eltex P HV 001.

Le mélange est ensuite extrudé à 180°C au moyen d'une extrudeuse bivis ZSK 30 (commercialisée par la Société Wermer et Pfeiderer).

Les granulés obtenus sont ensuite injectés à 220°C au moyen d'une presse à injecter Arburg 350-90-220 D avec un cycle de 41 secondes. Le moule est maintenu à la température de 35°C.

On obtient ainsi une éprouvette parallépipèdique à double épaisseur (2 mm et 4 mm) présentant une largeur de 49 mm et une longueur de 69 mm. Cette éprouvette présente une très belle et régulière couleur rouge.

Les coordonnées chromatiques de cette éprouvette, mesurées sur la partie épaisse de cette dernière (4 mm), sont alors les suivantes :
- L* = 38
- a* = 42,5
- b* = 32,5

### EXEMPLE 8

Cet exemple illustre l'utilisation d'un produit de l'invention dans la préparation d'un rouge à lèvres.

Les éléments constitutifs du rouge à lèvres sont donnés ci-dessous :

| **CONSTITUANTS** | **% EN POIDS** |
|---|---|
| **A** | |
| Silbione huile 70641 V 2OO | 40,5 |
| **B** | |
| Cire d'abeille | 7,5 |
| Cire de carnauba | 3,5 |
| Ozocérite | 3,5 |
| Paraffine | 10,0 |
| Huile de vaseline | q.s.p. 100 |
| Triglycéride d'acide caprique/caprylique (*) | 20,0 |
| **C** | |
| Pigments selon exemple 2 | q.s. |
| Oxyde de titane | q.s. |

| | |
|---|---|
| **(*) MYRITOL 318 de HENKEL** | |

Les éléments du mélange B sont fondus à une température de 85 ± 2°C, puis maintenus au bain thermostaté régulé à 60 ± 2°C.

Le pigment et l'oxyde de titane sont malaxés avec l'huile SILBIONE; ce mélange A-C est placé au bain thermostaté (60 ± 2°C).

Le mélange B est ensuite ajouté.

L'ensemble est coulé dans un moule siliconé.

## Revendications

1. Compositions caractérisées par le fait qu'elles comprennent :
- un support à base d'au moins un sesquisulfure de terres rares de formule M₂S₃ dans laquelle M représente au moins un élément pris dans le groupe constitué par les lanthanides de numéro atomique compris inclusivement entre 57 et 71 et l'yttrium, ledit support contenant en outre au moins un élément alcalin et/ou alcalino-terreux (élément dopant) dont une partie au moins est incluse dans le réseau cristallin dudit sesquisulfure de terres rares,
- et une couche à base d'au moins un oxyde transparent déposée à la surface dudit support et enrobant ce dernier.

2. Compositions selon la revendication 1, caractérisées en ce que ledit oxyde transparent est choisi, seul ou en mélange, dans le groupe constitué par la silice, l'alumine, la zircone, le zircon, l'oxyde de titane et les oxydes de terres rares.

3. Compositions selon l'une des revendications 1 ou 2, caractérisées en ce que ladite couche est à base de silice.

4. Compositions selon la revendication 3, caractérisées en ce que ladite couche est uniquement constituée de silice.

5. Compositions selon l'une quelconque des revendications précédentes, caractérisées en ce que ledit élément dopant dans ledit support est essentiellement présent sous une forme incluse dans le réseau cristallin dudit sesquisulfure.

6. Compositions selon l'une quelconque des revendications précédentes, caractérisées en ce que ledit élément dopant est présent dans les lacunes cationiques du réseau cristallin dudit sesquisulfure.

7. Compositions selon l'une quelconque des revendications précédentes, caractérisées en ce que la quantité molaire en élément dopant dans le support représente au moins 0,1% de la quantité molaire en élément M.

8. Compositions selon la revendication 7, caractérisées en ce que la quantité molaire en élément dopant dans le support est comprise entre 5 et 50% de la quantité molaire en élément M.

9. Compositions selon l'une quelconque des revendications précédentes, caractérisées en ce que ledit élément dopant est choisi, seul ou en mélange, parmi les éléments alcalins.

10. Compositions selon la revendication 9, caractérisées en ce que ledit élément dopant est le sodium.

11. Compositions selon l'une quelconque des revendications précédentes, caractérisées en ce que ledit sesquisulfure de terres rares présente une phase cristalline de type Th₃P₄.

12. Compositions selon l'une quelconque des revendications précédentes, caractérisées en ce que ledit sesquisulfure de terres rares est le sesquisulfure de cérium Ce2S3 γ cubique.

13. Compositions selon la revendication 12, caractérisées en ce qu'elles présentent une coordonnée chromatique L* au moins égale à 30.

14. Compositions selon la revendication 13, caractérisées en ce que ladite coordonnée chromatique L* est comprise entre 30 et 60.

15. Compositions selon l'une quelconque des revendications 12 à 14, caractérisées en ce qu'elles présentent une coordonnée chromatique a* au moins égale à 30.

16. Compositions selon la revendication 15, caractérisées en ce que ladite coordonnée chromatique a* est comprise entre 35 et 65.

17. Compositions selon l'une quelconque des revendications 12 à 16, caractérisées en ce qu'elles présentent une coordonnée chromatique b* comprise entre 0 et 40.

18. Compositions selon l'une quelconque des revendications précédentes, caractérisées en ce qu'elles présentent une granulométrie moyenne comprise entre 1 et 5 µm.

19. Compositions selon l'une quelconque des revendications précédentes, caractérisées en ce qu'elles sont constituées d'un support en sesquisulfure de cérium dopé par du sodium qui est enrobé par une couche homogène de silice transparente.

20. Compositions selon l'une quelconque des revendications précédentes, caractérisées en ce que le support présente un diamètre moyen de grain compris entre 0,2 et 5µm.

21. Procédé de préparation de compositions tels que définies à l'une quelconque des revendications précédentes, caractérisé en ce qu'il comprend les étapes suivantes :
(i) on met en contact le support tel que ci-avant défini et un précurseur de l'oxyde transparent désiré,
(ii) on précipite l'oxyde transparent,
(iii) et enfin on sépare la composition obtenue du milieu de réaction.

22. Procédé selon la revendication 21, caractérisé en ce que l'on procède à la précipitation de l'oxyde transparent désiré par hydrolyse d'un précurseur de type alcoolate.

23. Procédé selon la revendication 21, caractérisé en ce que l'on met en oeuvre un alcoolate de silicium, d'aluminium, de titane, de zirconium et/ou de terres rares.

24. Procédé selon la revendication 21, caractérisé en ce que l'on procède à la précipitation de l'oxyde transparent désiré par réaction entre un précurseur de type sel soluble et une base.

25. Procédé selon la revendication 24, caractérisé en ce que ladite base est choisie parmi l'ammoniaque et la soude.

26. Procédé selon l'une des revendications 24 et 25, caractérisé en ce que ledit sel soluble est choisi parmi les halogénures, en particulier les chlorures, et les nitrates.

27. Procédé selon l'une quelconque des revendications 24 à 26, caractérisé en ce que l'on met en oeuvre des sels solubles de silicium, d'aluminium, de zirconium, de titane et/ou de terres rares.

28. Procédé selon l'une des revendications 24 à 26, caractérisé en ce que ledit sel soluble et ladite base sont introduits de manière simultanée dans une suspension du support.

29. Procédé selon la revendication 21 pour l'obtention d'une composition comprenant une couche enrobante à base de silice, respectivement d'alumine, caractérisé en ce que l'on procède à la précipitation par réaction entre un précurseur de type silicate, respectivement de type aluminate, et un acide.

30. Procédé selon la revendication 29, caractérisé en ce que l'on utilise des silicates, respectivement des aluminates, d'alcalins.

31. Procédé selon l'une quelconque des revendications 29 et 30, caractérisé en ce que ledit acide est choisi parmi l'acide sulfurique, l'acide chlorhydrique et l'acide nitrique.

32. Procédé selon l'une quelconque des revendications 29 à 31, caractérisé en ce que ledit silicate, respectivement ledit aluminate, et ledit acide sont introduits de manière simultanée dans une suspension du support.

33. Procédé selon l'une quelconque des revendications 29 à 31, caractérisé en ce que ledit acide est introduit dans une solution de silicate, respectivement d'aluminate, contenant en suspension ledit support.

34. Procédé selon la revendication 31 pour l'obtention d'une composition comprenant une couche enrobante à base de zircon, caractérisé en ce que l'on co-hydrolyse, dans une suspension du support contenant en outre du fluorure de sodium,, un alcoolate de zirconium d'une part et un alcoolate de silicium d'autre part, puis on calcine la composition récupérée.

35. Pigments colorés, caractérisés en ce qu'ils sont à base de compositions selon l'une des revendications 1 à 20.

36. Utilisation des compositions définies à l'une quelconque des revendications 1 à 20, ou des pigments selon la revendication 35 dans des matières plastiques, des peintures, des lasures, des caoutchoucs, des céramiques, des glaçures, des papiers, des encres, des produits cosmétiques, des teintures et des revêtements stratifiés.

37. Compositions de matière colorées notamment du type plastiques, peintures, lasures, caoutchoucs, céramiques, glaçures, papiers, encres, produits cosmétiques, teintures et revêtement stratifiés, caractérisées en ce qu'elles comprennent des compositions telles que définies à l'une quelconque des revendications 1 à 20 ou des pigments selon la revendication 35.

## Patentansprüche

1. Zusammensetzungen, dadurch gekennzeichnet, daß sie umfassen:
- einen Träger auf der Basis von mindestens einem Sesquisulfid der seltenen Erden mit der Formel M₂S₃, worin M mindestens ein aus der Gruppe gewähltes Element darstellt, die aus den Lanthaniden der Atomnummern zwischen einschließlich 57 und 71 und Yttrium gebildet wird, wobei der genannte Träger außerdem mindestens ein Alkali- und/oder Erdalkalimetall-Element (Dotierungs-Element) enthält, von dem mindestens ein Teil in dem Kristallgitter des genannten Sesquisulfids der seltenen Erden eingeschlossen ist, und
- eine Schicht auf der Basis von mindestens einem transparenten Oxid, das auf der Oberfläche des genannten Trägers abgelagert ist und diesen umhüllt.

2. Zusammensetzungen nach Anspruch 1, dadurch gekennzeichnet, daß das genannte transparente Oxid, allein oder in Mischung, aus der durch Siliciumdioxid, Aluminiumoxid, Zirkoniumdioxid, Zirkonium, Titanoxid und die Oxide der seltenen Erden gebildeten Gruppe ausgewählt wird.

3. Zusammensetzungen nach einem der Ansprüche 1 oder 2, dadurch gekennzeichnet, daß die genannte Schicht auf der Basis von Siliciumdioxid gebildet wird.

4. Zusammensetzungen nach Anspruch 3, dadurch gekennzeichnet, daß die genannte Schicht nur aus Siliciumdioxid besteht.

5. Zusammensetzungen nach irgendeinem der vorstehenden Ansprüche, dadurch gekennzeichnet, daß das genannte Dotierungs-Element, das in dem genannten Träger notwendigerweise anwesend ist, in Form eines Einschlusses in dem kristallinen Gitter des genannten Sesquisulfids vorliegt.

6. Zusammensetzungen nach irgendeinem der vorstehenden Ansprüche, dadurch gekennzeichnet, daß das genannte Dotierungs-Element in den kationischen Gitterleerstellen des kristallinen Gitters des genannten Sesquisulfids anwesend ist.

7. Zusammensetzungen nach irgendeinem der vorstehenden Ansprüche, dadurch gekennzeichnet, daß die molare Menge des Dotierungs-Elementes in dem Träger mindestens 0,1 % der molaren Menge des Elementes M darstellt.

8. Zusammensetzungen nach Anspruch 7, dadurch gekennzeichnet, daß die molare Menge des Dotierungs-Elementes in dem Träger zwischen 5 und 50 % der molaren Menge des Elementes M beträgt.

9. Zusammensetzungen nach irgendeinem der vorstehenden Ansprüche, dadurch gekennzeichnet, daß das Dotierungs-Element, allein oder in Mischung, unter den Alkalimetall-Elementen ausgewählt wird.

10. Zusammensetzungen nach Anspruch 9, dadurch gekennzeichnet, daß das genannte Dotierungs-Element Natrium ist.

11. Zusammensetzungen nach irgendeinem der vorstehenden Ansprüche, dadurch gekennzeichnet, daß das genannte Sesquisulfid der seltenen Erden eine kristalline Phase vom Typ Th₃P₄ aufweist.

12. Zusammensetzungen nach irgendeinem der vorstehenden Ansprüche, dadurch gekennzeichnet, daß das genannte Sesquisulfid der seltenen Erden das Sesquisulfid von Cer Ce₂S₃ γ kubisch ist.

13. Zusammensetzungen nach Anspruch 12, dadurch gekennzeichnet, daß sie eine chromatische Koordinate L" von mindestens gleich 30 aufweisen.

14. Zusammensetzungen nach Anspruch 13, dadurch gekennzeichnet, daß die genannte chromatische Koordinate L" zwischen 30 und 60 beträgt.

15. Zusammensetzungen nach irgendeinem der Ansprüche 12 bis 14, dadurch gekennzeichnet, daß sie eine chromatische Koordinate a" von mindestens gleich 30 aufweisen.

16. Zusammensetzungen nach Anspruch 15, dadurch gekennzeichnet, daß die genannte chromatische Koordinate a zwischen 35 und 65 beträgt.

17. Zusammensetzungen nach irgendeinem der Ansprüche 12 bis 16, dadurch gekennzeichnet, daß sie eine chromatische Koordinate b" zwischen 0 und 40 aufweisen.

18. Zusammensetzungen nach irgendeinem der vorstehenden Ansprüche, dadurch gekennzeichnet, daß sie eine mittlere Granulometrie zwischen 1 und 5 µm aufweisen.

19. Zusammensetzungen nach irgendeinem der vorstehenden Ansprüche, dadurch gekennzeichnet, daß sie von einem mit Natrium dotierten Träger aus Cer-Sesquisulfid gebildet werden, der mit einer homogenen Schicht aus transparentem Siliciumdioxid umhüllt ist.

20. Zusammensetzungen nach irgendeinem der vorstehenden Ansprüche, dadurch gekennzeichnet, daß der Träger einen mittleren Korndurchmesser zwischen 0,2 und 5 µm aufweist.

21. Verfahren zur Herstellung der wie in irgendeinem der vorstehenden Ansprüche definierten Zusammensetzungen, dadurch gekennzeichnet, daß es die folgenden Stufen umfaßt:
(i) man bringt den wie oben definierten Träger und einen Vorläufer des gewünschten transparenten Oxids in Kontakt,
(ii) man fällt das transparente Oxid, und
(iii) man trennt schließlich die erhaltene Zusammensetzung von dem Reaktionsmedium ab.

22. Verfahren nach Anspruch 21, dadurch gekennzeichnet, daß man die Fällung des gewünschten transparenten Oxids durch Hydrolyse eines Vorläufers vom Typ Alkoholat vornimmt.

23. Verfahren nach Anspruch 21, dadurch gekennzeichnet, daß man ein Alkoholat von Silicium, Aluminium, Titan, Zirkonium und/oder der seltenen Erden einsetzt.

24. Verfahren nach Anspruch 21, dadurch gekennzeichnet, daß man die Fällung des gewünschten transparenten Oxids durch Reaktion zwischen einem Vorläufer vom Typ eines löslichen Salzes und einer Base vornimmt.

25. Verfahren nach Anspruch 24, dadurch gekennzeichnet, daß die genannte Base unter Ammoniak und Natriumhydroxid ausgewählt wird.

26. Verfahren nach einem der Ansprüche 24 und 25, dadurch gekennzeichnet, daß das genannte lösliche Salz unter den Halogeniden, insbesondere den Chloriden, und den Nitraten ausgewählt wird.

27. Verfahren nach irgendeinem der Ansprüche 24 bis 26, dadurch gekennzeichnet, daß man lösliche Salze von Silicium, Aluminium, Titan, Zirkonium und/oder der seltenen Erden einsetzt.

28. Verfahren nach einem der Ansprüche 24 bis 26, dadurch gekennzeichnet, daß das genannte lösliche Salz und die genannte Base gleichzeitig in eine Suspension des Trägers eingebracht werden.

29. Verfahren nach Anspruch 21 zur Herstellung einer Zusammensetzung, die eine umhüllende Schicht auf der Basis von Siliciumdioxid bzw. Aluminiumoxid umfaßt, dadurch gekennzeichnet, daß man die Fällung durch Reaktion zwischen einem Vorläufer vom Typ Silikat bzw. Aluminat und einer Säure vornimmt.

30. Verfahren nach Anspruch 29, dadurch gekennzeichnet, daß man Silikate bzw. Aluminate von Alkalimetallen verwendet.

31. Verfahren nach irgendeinem der Ansprüche 29 und 30, dadurch gekennzeichnet, daß die genannte Säure unter Schwefelsäure, Chlorwasserstoffsäure und Salpetersäure ausgewählt wird.

32. Verfahren nach irgendeinem der Ansprüche 29 bis 31, dadurch gekennzeichnet, daß das genannte Silikat bzw. Aluminat und die genannte Säure gleichzeitig in eine Suspension des Trägers eingebracht werden.

33. Verfahren nach irgendeinem der Ansprüche 29 bis 31, dadurch gekennzeichnet, daß die genannte Säure in eine Lösung des genannte Silikats bzw. Aluminats eingebracht wird, die in Suspension den genannten Träger enthält.

34. Verfahren nach Anspruch 21 zur Herstellung einer Zusammensetzung, die eine umhüllende Schicht auf der Basis von Zirkonium umfaßt, dadurch gekennzeichnet, daß man in einer Suspension des Trägers, die außerdem Natriumfluorid enthält, einerseits ein Zirkoniumalkoholat und andererseits ein Siliciumalkoholat co-hydrolysiert und anschließend die gewonnene Zusammensetzung kalziniert.

35. Farbige Pigmente, dadurch gekennzeichnet, daß sie auf der Basis der Zusammensetzungen nach einem der Ansprüche 1 bis 20 gebildet werden.

36. Verwendung der wie in irgendeinem der Ansprüche 1 bis 20 definierten Zusammensetzungen oder wie in Anspruch 35 definierten Pigmente in Kunststoffen, Anstrichstoffen, Lasuren, Kautschuks, Keramiken, Glasuren, Papieren, Tinten, kosmetischen Produkten, Färbemitteln und Schichtstoff-Überzügen.

37. Zusammensetzungen farbiger Stoffe, insbesondere vom Typ der Kunststoffe, Anstrichstoffe, Lasuren, Kautschuks, Keramiken, Glasuren, Papiere, Tinten, kosmetischen Produkte, Färbemittel und Schichtstoff-Überzüge, dadurch gekennzeichnet, daß sie Zusammensetzungen wie in irgendeinem der Ansprüche 1 bis 20 definiert oder Pigmente wie in Anspruch 35 definiert umfassen.

## Claims

1. Compositions characterised in that they comprise:
- a support based on at least one sesquisulphide of rare earths of the fomula M₂S₃ in which M represents at least one element selected from the group formed by lanthanides with an atomic number between 57 and 71 inclusive and yttrium, said support also containing at least one alkali metal and/or alkaline-earth element (doping element) of which a part at least is included in the crystalline lattice of said sesquisulphide of rare earths, and
- a layer based on at least one transparent oxide deposited at the surface of said support and covering the support.

2. Compositions according to claim 1 characterised in that said transparent oxide is selected, alone or in a mixture, from the group formed by silica, alumina, zirconia, zircon, titanium oxide and oxides of rare earths.

3. Compositions according to one of claims 1 and 2 characterised in that said layer is based on silica.

4. Compositions according to claim 3 characterised in that said layer is formed solely of silica.

5. Compositions according to any one of the preceding claims characterised in that said doping element in said support is essentially present in a form included in the crystalline lattice of said sesquisulphide.

6. Compositions according to any one of the preceding claims characterised in that said doping element is present in the cationic gaps in the crystalline lattice of said sesquisulphide.

7. Compositions according to any one of the preceding claims characterised in that the molar amount of doping element in the support represents at least 0.1% of the molar amount of element M.

8. Compositions according to claim 7 characterised in that the molar amount of doping element in the support is between 5 and 50% of the molar amount of element M.

9. Compositions according to any one of the preceding claims characterised in that said doping element is selected, alone or in a mixture, from alkali metal elements.

10. Compositions according to claim 9 characterised in that said doping element is sodium.

11. Compositions according to any one of the preceding claims characterised in that said sesquisulphide of rare earths has a crystalline phase of type Th₃P₄.

12. Compositions according to any one of the preceding claims characterised in that said sesquisulphide of rare earths is γ cubic cerium sesquisulphide Ce2S3.

13. Compositions according to claim 12 characterised in that they have a chromatic coordinate L* at least equal to 30.

14. Compositions according to claim 13 characterised in that said chromatic coordinate L* is between 30 and 60.

15. Compositions according to any one of claims 12 to 14 characterised in that they have a chromatic coordinate a* at least equal to 30.

16. Compositions according to claim 15 characterised in that said chromatic coordinate a* is between 35 and 65.

17. Compositions according to any one of claims 12 to 16 characterised in that they have a chromatic coordinate b* of between 0 and 40.

18. Compositions according to any one of the preceding claims characterised in that they have a mean granulometry of between 1 and 5 µm.

19. Compositions according to any one of the preceding claims characterised in that they are formed by a support of cerium sesquisulphide doped with sodium which is encased by a homogenous layer of transparent silica.

20. Compositions according to any one of the preceding claims characterised in that the support has a mean grain diameter of between 0.2 and 5 µm.

21. A process for the preparation of compositions as defined in any one of the preceding claims characterised in that it comprises the following steps:
(i) the support as defined hereinbefore and a precursor of the desired transparent oxide are brought into contact,
(ii) the transparent oxide is precipitated, and
(iii) finally the composition obtained is separated from the reaction medium.

22. A process according to claim 21 characterised in that precipitation of the desired transparent oxide is effected by hydrolysis of a precursor of alcoholate type.

23. A process according to claim 21 characterised by using an alcoholate of silicon, aluminium, titanium, zirconium and/or rare earths.

24. A process according to claim 21 characterised by effecting precipitation of the desired transparent oxide by reaction between a precursor of soluble salt type and a base.

25. A process according to claim 24 characterised in that said base is selected from ammonia and sodium hydroxide.

26. A process according to one of claims 24 and 25 characterised in that said soluble salt is selected from halides, in particular chlorides, and nitrates.

27. A process according to any one of claims 24 to 26 characterised by using soluble salts of silicon, aluminium, zirconium, titanium and/or rare earths.

28. A process according to one of claims 24 to 26 characterised in that said soluble salt and said base are introduced simultaneously into a suspension of the support.

29. A process according to claim 21 for producing a composition comprising an encasing layer based on silica or alumina respectively, characterised by effecting precipitation by reaction between a precursor of silicate type or aluminate type respectively and an acid.

30. A process according to claim 29 characterised by using silicates or aluminates respectively of alkali metals.

31. A process according to either one of claims 29 and 30 characterised in that said acid is selected from sulphuric acid, hydrochloric acid and nitric acid.

32. A process according to any one of claims 29 to 31 characterised in that said silicate or said aluminate respectively and said acid are introduced simultaneously into a suspension of the support.

33. A process according to any one of claims 29 to 31 characterised in that said acid is introduced into a solution of silicate or aluminate respectively containing said support in suspension.

34. A process according to claim 31 for producing a composition comprising an encasing layer based on zircon characterised by co-hydrolyising in a suspension of the support also containing sodium fluorate, a zirconium alcoholate on the one hand and a silicon alcoholate on the other hand and then calcining the composition recovered.

35. Coloured pigments characterised in that they are based on compositions according to one of claims 1 to 20.

36. Use of the compositions defined in any one of claims 1 to 20 or the pigments according to claim 35 in plastics materials, paints, varnishes, rubbers, ceramics, glazes, papers, inks, cosmetic products, dyes and laminated coatings.

37. Coloured compositions of material in particular of the plastics, paints, varnishes, rubbers, ceramics, glazes, papers, inks, cosmetic products, dyes and laminated coatings type, characterised in that they comprise compositions as defined in any one of claims 1 to 20 or pigments according to claim 35.
